# EUROPEAN PATENT SPECIFICATION

(11) **EP 2 235 052 B1**
(45) Date of publication and mention of the grant of the patent: **20.11.2013**
(21) Application number: 09703503.4
(22) Date of filing: 21.01.2009
(51) Int. Cl.: C07K 14/435, C07K 16/46

(54) **PROTEINACEOUS BINDING MOLECULES COMPRISING PURIFICATION TAGS**
PROTEINÖSE BINDEMOLEKÜLE MIT REINIGUNGSTAGS
MOLÉCULES À LIAISON PROTÉIQUE COMPRENANT DES ÉTIQUETTES DE PURIFICATION

(30) Priority: 21.01.2008 EP 08100707; 22.01.2008 US 22661 P; 22.04.2008 US 46957 P
(43) Date of publication of application: 06.10.2010
(73) Proprietor: MorphoSys AG, 82152 Planegg-Martinsried (DE)
(72) Inventor: STEIDL, Stefan, 81241 München (DE)
(74) Representative: Hutter, Bernd
(86) International application number: PCT/EP2009/050656
(87) International publication number: WO 2009/092732

(56) References cited:
- BORTOLETTO NICOLA ET AL: "Optimizing anti-CD3 affinity for effective T cell targeting against tumor cells" EUROPEAN JOURNAL OF IMMUNOLOGY, WEINHEIM, DE, vol. 32, no. 11, November 2002 (2002-11), pages 3102-3107, XP002436763 ISSN: 0014-2980
- SCHIWECK WOLFRAM ET AL: "Sequence analysis and bacterial production of the anti-c-myc antibody 9E10: The V-H domain has an extended CDR-H3 and exhibits unusual solubility" FEBS LETTERS, vol. 414, no. 1, 1997, pages 33-38, XP002477221 ISSN: 0014-5793
- MULLER K M ET AL: "A dimeric bispecific miniantibody combines two specificities with avidity" FEBS LETTERS, ELSEVIER, AMSTERDAM, NL, vol. 432, no. 1-2, 31 July 1998 (1998-07-31), pages 45-49, XP004259191 ISSN: 0014-5793
- PADIOLLEAU-LEFEVRE ET AL: "Expression and detection strategies for an scFv fragment retaining the same high affinity than Fab and whole antibody: Implications for therapeutic use in prion diseases" MOLECULAR IMMUNOLOGY, ELMSFORD, NY, US, vol. 44, no. 8, 1 December 2006 (2006-12-01), pages 1888-1896, XP005792725 ISSN: 0161-5890
- LADNER ROBERT C ET AL: "Novel frameworks as a source of high-affinity ligands" CURRENT OPINION IN BIOTECHNOLOGY, vol. 12, no. 4, August 2001 (2001-08), pages 406-410, XP002524558 ISSN: 0958-1669
- NUTTALL S D ET AL: "DESIGN AND EXPRESSION OF SOLUBLE CTLA-4 VARIABLE DOMAIN AS A SCAFFOLD FOR THE DISPLAY OF FUNCTIONAL POLYPEPTIDES" PROTEINS: STRUCTURE, FUNCTION AND GENETICS, JOHN WILEY & SONS, INC, US, vol. 36, no. 2, 1 January 1999 (1999-01-01), pages 217-227, XP000866035 ISSN: 0887-3585
- GOEL A ET AL: "Relative position of the hexahistidine tag effects binding properties of a tumor-associated single-chain Fv construct" BIOCHIMICA ET BIOPHYSICA ACTA - GENERAL SUBJECTS, ELSEVIER SCIENCE PUBLISHERS, NL, vol. 1523, no. 1, 1 September 2000 (2000-09-01), pages 13-20, XP004276666 ISSN: 0304-4165
- TODOROVSKA ANETA ET AL: "Design and application of diabodies, triabodies and tetrabodies for cancer targeting" JOURNAL OF IMMUNOLOGICAL METHODS, ELSEVIER SCIENCE PUBLISHERS B.V.,AMSTERDAM, NL, vol. 248, no. 1-2, 1 February 2001 (2001-02-01), pages 47-66, XP002288731 ISSN: 0022-1759
- PLUECKTHUN A ET AL: "NEW PROTEIN ENGINEERING APPROACHES TO MULTIVALENT AND BISPECIFIC ANTIBODY FRAGMENTS" IMMUNOTECHNOLOGY, ELSEVIER SCIENCE PUBLISHERS BV, NL, vol. 3, 1997, pages 83-105, XP002950976 ISSN: 1380-2933

## Description

### Background of the invention

Immunoglobulins, such as antibodies, are of continued and increasing interest for the pharmaceutical industry. More than 20 monoclonal antibodies (mAb's) are on the market and more than 100 in clinical trials. In the last decade, numerous derivatives and fragments of immunoglobulins were designed that have certain different properties compared to conventional immunoglobulins and antibodies, which may be desirable for certain diagnostic and therapeutic applications. Examples are recombinant antibody fragments (such as classic monovalent antibody fragments (Fab, scFv)) or engineered variants (diabodies, triabodies, minibodies and single-domain antibodies), some of which are now emerging as credible alternatives. Such fragments and variants usually retain the target specificity of full-length immunoglobulins but can often be produced more economically. In addition, new 'antibody-like' protein display scaffolds were developed, which, likewise, possess certain other properties compared to conventional immunoglobulins and antibodies.

For certain therapeutic applications, it would be desirable to have a monovalent antibody-antigen interaction, i.e. an immunoglobulin, or another scaffold, with only one variable domain that binds to a target molecule of interest. Some IgGs have been found to crosslink target receptors and thereby mimic the effect of the natural ligand. Cross-linking can lead to receptor activation (e.g. receptor phosphorylation). In contrast, monovalent Fab's derived from the same antibody do not lead to receptor cross-linking and, if the appropriate epitope is targeted, prevent the natural ligand from binding. Thus, while the IgG acts agonistic, the Fab fragment of the same antibody acts antagonistic. Examples are the insulin receptor (Kahn et al., Proc Natl Acad Sci U S A. (1978) 75:4209-13), the EGF receptor (Schreiber et al., J Biol Chem. (1983) 258:846-53), the EPO receptor (Schneider et al, Blood (1997) 89:473-82), the GH receptor (Wan et al., Mol Endocrinol. (2003) 17:2240-50) or the beta2-Adrenoceptor (Mijares et al., Mol Pharmacol. (2000) 58:373-9).

However, constructs with such monovalent properties, e.g. Fab fragments, may be disadvantageous, due to other properties, such as a short half-life in vivo. It would, therefore, be highly desirable to combine a monovalent antibody-antigen interaction with the properties of a full-length immunoglobulin comprising the Fc domain. Such molecules are provided in the present invention.

### Summary of the invention

It is an object of the present invention to provide an immunoglobulin comprising at least two variable domains, wherein a variable domain includes the entire antigen-binding region of one arm of the immunoglobulin molecule, wherein one variable domain binds to a target molecule, and wherein at least one other variable domain comprises a purification tag, wherein the variable domain comprising the purification tag does not bind to the target molecule, and wherein said purification tag is comprised in the CDR region of said other variable domain and is selected from a histidine tag, a hexahistidine tag, a Strep tag, a myc tag, a Flag tag and a V5 tag, or a substantially identical variant thereof which retains the property of the purification tag of specifically binding to a moiety which specifically recognizes the purification tag and wherein the immunoglobulin is monovalent with respect to binding of the target molecule but retains the properties of a full-length immunoglobulin in respect of size and presence of the Fc domain.

In one embodiment, said purification tag is comprised in the H-CDR3 or the H-CDR2.

In another embodiment, said purification tag is comprised in the L-CDR2.

It is an object of the present invention to provide a nucleic acid sequence that encodes an immunoglobulin comprising at least two variable domains, wherein a variable domain includes the entire antigen-binding region of one arm of the immunoglobulin molecule, wherein one variable domain binds to a target molecule, and wherein at least one other variable domain comprises a purification tag.

In another embodiment the present invention provides a vector comprising said nucleic acid sequence.

In another embodiment the present invention provides a cell comprising said vector. In a further embodiment said cell is a mammalian cell.

It is an object of the present invention to provide a pharmaceutical composition comprising an immunoglobulin of the present invention.

In one embodiment said immunoglobulin is used in the diagnosis of a disease or disorder.

In one embodiment said immunoglobulin is used in the treatment of a disease or disorder.

It is an object of the present invention to provide a method to purify said immunoglobulin, comprising the steps of (a) providing a solution comprising an immunoglobulin according to any one of claims 1 to 3, (b) bringing the solution of step (a) in contact with a moiety with affinity to the purification tag of said immunoglobulin, (c) allowing the binding of the immunoglobulin to said moiety, (d) removing all or substantially all constituents of the solution which do not bind to the moiety, and (e) recovering the immunoglobulin from the moiety.

### Brief description of the figures

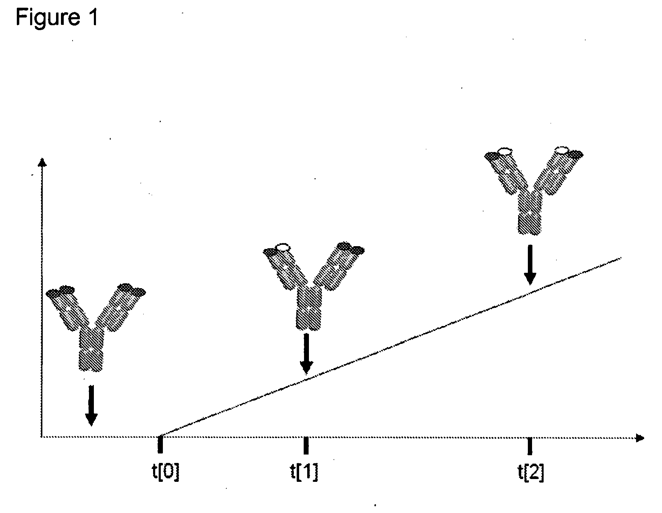

Figure 1 depicts the purification of proteinaceous binding molecules of the present invention via Ni-NTA chromatography.
   The x-axis is the time axis. The y-axis indicates the concentration of the ingredient used to elute the material from the column (in the present case imidazole). 'Filled circles' indicate that the respective VH or VL does not comprise a purification tag. When such a VH is combined with a VL which also does not comprise a purification tag the respective arm is specific for a target molecule. 'Open circles' indicate that at least one CDR of the respective VH or VL comprises a purification tag. The three different immunoglobulin species elute at different fractions in an imidazole gradient. The immunoglobulin species without a purification tag is found in the flow through fractions, and the immunoglobulin species comprising a purification tag on one arm of the molecule is eluted earlier compared to the immunoglobulin species comprising a purification tag on both arms of the molecule.

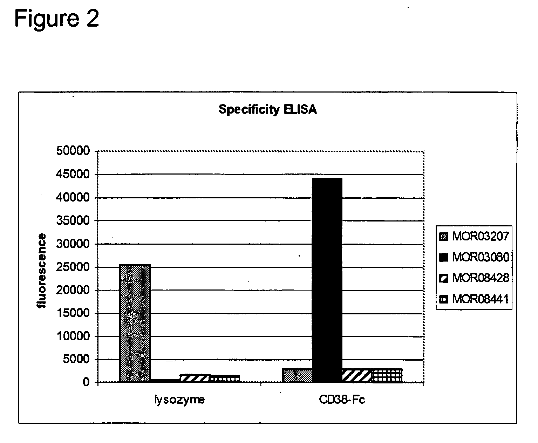

Figure 2 shows binding specificity of four Fab fragments as analysed by ELISA. Either hen egg lysozyme or CD38-Fc was coated (5µg/ml) in a Maxisorp ELISA plate (Nunc). Fab fragments were added and after washing specifically bound Fab fragments were detected by incubation with AP conjugated anti-human IgG (Fab)₂ specific antibody followed by development with soluble Atto-Phos substrate (Roche) diluted in water. Fluorescence measurements were performed in a Tecan plate reader at 535 nm emission. Fabs tested were MOR03207 (hen egg lysozyme specific), MOR03080 (CD38 specific), MOR08428 (H-CDR3 of MOR03207 was replaced by GYSGHHHHHHSGDY) and MOR08441 (VH of MOR08428 and VL of MOR03080).

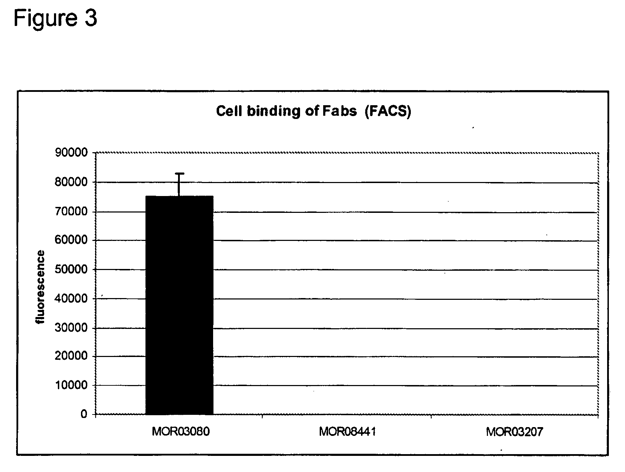

Figure 3 shows binding specificity of three Fab fragments as analysed by FACS using CHO-K1 cells recombinantly expressing human CD38. Fab fragments were added to the cells and after washing, specifically bound Fab fragments were detected by incubation with PE conjugated anti-human IgG (Fab)₂ specific antibody (Jackson Immuno Research). Median fluorescence was determined with a FACSArray instrument (Becton Dickinson). Fabs tested were MOR03207 (hen egg lysozyme specific), MOR03080 (CD38 specific) and MOR08441 (VH of MOR08428 and VL of MOR03080).

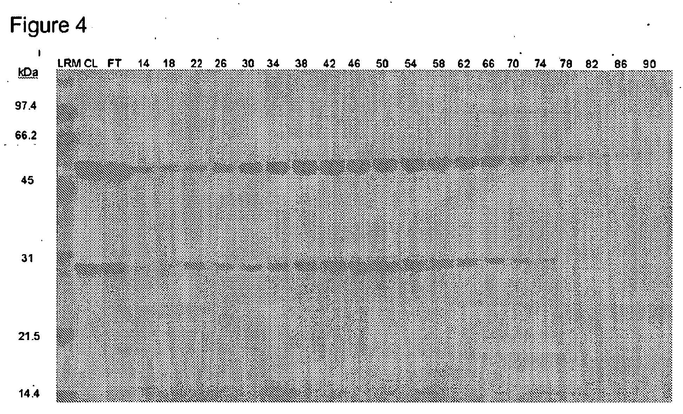

Figure 4 shows a reducing SDS-PAGE of protein fractions obtained from IMAC purification using a His-Bind Fractogel (Merck #70693-3) column applying an imiazole gradient for elution. Protein A column pre-purified IgG1 fractions obtained after cotransfection of pMorph2_h_IgG1_MOR08428, pMorph2_h_IgG1_MOR03080, and pMorph2_h_Ig_lambda2_MOR03080 were loaded on a reducing SDS gel. At a flow-rate of 1 ml/min 1 ml fractions were taken. As running buffer 20 mM Na-phosphate pH 7.4/500 mM NaCl/10 mM imidazole was used. As elution buffer running buffer + 250 mM imidazole was used, while a gradient (0 - 100%) of elution buffer was applied.
   LRM: Protein size markers; CL: Pre-purified IgG1 fractions loaded onto the column ;
   FT: Column flow through; Numbers indicate different elution fractions.

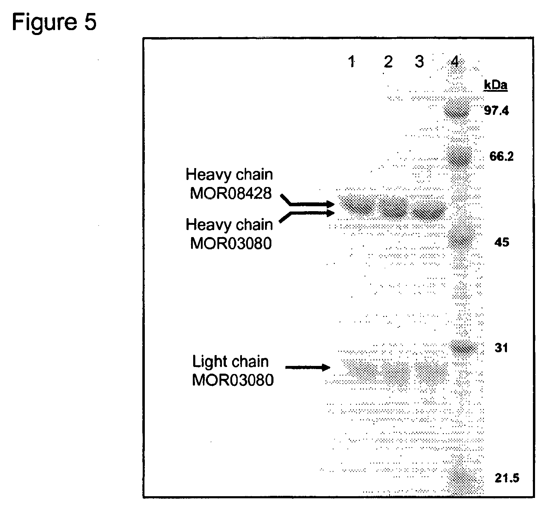

Figure 5 shows a reducing SDS-PAGE of protein fractions obtained from the affinity chromatography flow through (lane 3) and the samples from pooled fractions #30-58 (lane 2) and #70-90 (lane 1). Lane 4 was loaded with protein size markers. For details see Example 3.

### Detailed description of the invention

The present disclosure provides proteinaceous binding molecules, such as immunogloblins, with superior advantages over known molecules in the art. Often, immunoglobulins lead to the cross-linking of the respective targets due to the bivalent character of these molecules. Also other constructs known in the art, such as e.g. bispecific molecules resulting from a gene fusion of scFv fragments, as disclosed in Mueller et al., 1998; FEBS Letters, also induce cross-linking by its bivalent structure. Such cross-linking activity can lead to several unwanted effects in a particular application, for example receptor activation (e.g. via receptor phosphoylation).

On the other hand, there is very often a need for full length immunoglobulins. This need may be due to several reasons, such as the need for molecules with a longer half life (Fab molecules, for example, have a very short half life compared to imunoglobulins) or the need for molecules with all properties of an immunoglobulin, especially full length immunoglobulins (effector function, as one example).

This apparent dilemma is solved by the present inventions. The proteinaceous binding molecules described herein are monovalent in nature, but comprise all the properties of a full-length immunoglobulin. Furthermore, by the way the proteinaceous binding molecules are constructed, the present invention also provides methods which enable the convenient purification of these molecules.

As used herein, a molecule, such as a proteinaceous binding molecule, "binds specifically to," is "specific to/for" or "specifically recognizes" a target molecule, such as an antigen, if such proteinaceous binding molecule is able to discriminate between such target molecule and one or more reference molecules(s), since binding specificity is not an absolute, but a relative property. In its most general form (and when no defined reference is mentioned), "specific binding" is referring to the ability of the proteinaceous binding molecule to discriminate between the target molecules of interest and an unrelated molecule, as determined, for example, in accordance with one of the following methods. Such methods comprise, but are not limited to Western blots, ELISA-, RIA-, ECL-, IRMA-tests and peptide scans. For example, a standard ELISA assay can be carried out. The scoring may be carried out by standard color development (e.g. secondary antibody with horseradish peroxide and tetramethyl benzidine with hydrogenperoxide). The reaction in certain wells is scored by the optical density, for example, at 450 nm. Typical background (=negative reaction) may be 0.1 OD; typical positive reaction may be 1 OD. This means the difference positive/negative can be more than 10-fold. Typically, determination of binding specificity is performed by using not a single reference molecule, but a set of about three to five unrelated molecules, such as milk powder, BSA, transferrin or the like.

However, "specific binding" also may refer to the ability of a proteinaceous binding molecule to discriminate between the target molecule and one or more closely related molecule(s), which are used as reference points, e.g. between target X and target Y. Additionally, "specific binding" may relate to the ability of a proteinaceous binding molecule to discriminate between different parts of its target molecule, e.g. different domains or regions of the target molecule, such as epitopes in the N-terminal or in the C-terminal region of the target molecule, or between one or more key amino acid residues or stretches of amino acid residues of the target molecule.

The term "proteinaceous binding molecule" as used herein refers to a molecule comprising at least two amino-acids in peptidic linkage with each other. The proteinaceous molecule is preferably a molecule that is produced by a biological organism or a part, derivative and/or analogue thereof. It is clear that derivatives generated through modification of the compound after the production by a biological molecule are also preferred compound of the invention. In a particularly preferred embodiment the proteinaceous binding molecules are immunoglubulins, such as antibodies, or fragments thereof. Other preferred proteinaceous binding molecules include scaffolds with antibody-like properties, such as affibodies (which comprise the Z-domain of protein A), immunity proteins (such as ImmE7), cytochrome b₅₆₂, proteins comprising ankyrin repeats, PDZ domains or Kunitz domains, insect defensin A, scorpion toxins (such as charybdotoxin or CTLA-4), knottins (such as Min-23, neocarzinostatin, CBM4-2 or tendamistat), anticalins or armadillo repeat proteins.

An "immunoglobulin" (Ig), as used herein, refers to a protein belonging to the class IgG, IgM, IgE, IgA, or IgD (or any subclass thereof), and includes all conventionally known antibodies and functional fragments thereof. A "functional fragment" of an antibody/immunoglobulin as defined herein refers to a fragment of an antibody/immunoglobulin (e.g., a variable region of an IgG) that comprises at least two variable domains in which the first variable domains retains the binding properties of the respective full-length immunoglobulin to the target molecule. Functional fragments of the invention include F(ab')2 fragments or Fab-dHLX fragments. Such fragments may be engineered to minimize or completely remove the intermolecular disulphide interactions that occur between the CH1 and CL domains. The term "functional fragment" of a proteinaceous binding molecule of the present invention refers to any proteinaceous moiety, in which wherein a first variable domain, but none of said other variable domains, binds to a target molecule of interest. In certain preferred embodiments, the immunoglobulin of the present invention is a full length immunoglobulin or substantially a full length immunoglobulin.

The term "variable domain" refers to the portion of a proteinaceous binding molecule which differs extensively in sequence between different proteinaceous binding molecules, and which confers binding and specificity of the proteinaceous binding molecule to a target molecule of interest (or lack thereof). In accordance with the present invention, peptide sequences, in particular purification tags as defined herein, are introduced into variable domains in a manner such that a variable domain does no longer specifically bind to a target molecule. Variable domains may have any shape or form. They may consist of polypeptide stretches comprised in one, two, or even more than two polypeptide chains. A variable domain of an immunoglobulin, such as an antibody, or a functional fragment thereof, typically comprises the entire antigen-binding region of one arm of the immunoglobulin molecule. It includes the CDR regions of both variable chains of one arm of the immunoglobulin molecule, i.e. the H-CDR3, the H-CDR2, the H-CDR1, the L-CDR3, the L-CDR2 and the L-CDR1, and the adjacent framework regions required for specific binding to a given target molecule.

An "antigen-binding region" of an antibody typically is found in one or more hypervariable region(s) of an antibody, i.e., the CDR-1, -2, and/or -3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320). A preferred class of immunoglobulins for use in the present invention is IgG.

According to the present invention, the first variable domain of a proteinaceous binding molecule binds to a target molecule. The other variable domains of the proteinaceous binding molecule have no binding specificity to the same target molecule. The proteinaceous binding molecule is, therefore, monovalent in respect of binding to the target molecule. Furthermore, at least one of the other variable domains, i.e. the variable domains which do not bind to the target molecule, comprises a purification tag suitable for purification of said proteinaceous binding molecule.

An antibody of the invention may be derived from a recombinant antibody library that is based on amino acid sequences that have been designed in silico and encoded by nucleic acids that are synthetically created. In silico design of an antibody sequence is achieved, for example, by analyzing a database of human sequences and devising a polypeptide sequence utilizing the data obtained

therefrom. Methods for designing and obtaining in silico-created sequences are described, for example, in Knappik et al., J. Mol. Biol. (2000) 296:57; Krebs et al., J. Immunol. Methods. (2001) 254:67; Rothe et al., J. Mol. Biol (2008) in press, and U.S. Patent No. 6,300,064 issued to Knappik et al., which hereby are incorporated by reference in their entirety.

"Target molecule" as refered to herein refers to any molecule of interest that binds to the first variable domain of the proteinaceous binding molecule. The target molecule refers to the molecule to which binding of the proteinaceous binding molecule is desired in order to achieve a certain biological effect and is to be discriminated from the binding partner of the second, or any subsequent, variable domain which comprises the purification tag, or which is inert. Typically and preferably the target molecule is a peptide, a protein or any other proteinaceous molecule. Alternatively the target molecules may be any other organic or inorganic molecules, such as carbohydrates, fatty acids, lipids, dyes or flourophors.

The term "purification tag" as used herein refers to a peptide sequence selected from a histidine tag, a hexahistidine tag, a Strep tag, a myc tag, a Flag tag and a V5 tag, or a substantially identical variant thereof which retains the property of the purification tag of specifically binding to a moiety which specifically recognizes the purification tag suitable for purification or identification of a proteinaceous binding molecule. The purification tag specifically binds to another moiety with affinity for the purification tag. Such moieties which specifically bind to a purification tag are usually attached to a matrix or a resin, such as agarose beads. Moieties which specifically bind to purification tags include antibodies, nickel or cobalt ions or resins, biotin, amylose, maltose, and cyclodextrin. Exemplary purification tags include histidine tags (such as a hexahistidine peptide), which will bind to metal ions such as nickel or cobalt ions. Therefore, in certain embodiments the purification tag comprises a peptide sequence which specifically binds metal ions. Exemplary purification tags are the myc tag (EQKLISEEDL), the Strep tag (WSHPQFEK), the Flag tag (DYKDDDDK) and the V5 tag (GKPIPNPLLGLDST). Exemplary the FLAG tag is specifically recognized by a monoclonal anti-FLAG antibody. The peptide sequence recognized by the anti-FLAG antibody consists of the sequence DYKDDDDK or a substantially identical variant thereof. Therefore, in certain embodiments the purification tag comprises or consists of a peptide sequence which is specifically recognized by an antibody. The term "purification tag" also includes substantially identical variants of purification tags. "Substantially identical variant" as used herein refers to derivatives or fragments of purification tags which are modified compared to the original purification tag (e.g. via amino acid substitutions, deletions or insertions), but which retain the property of the purification tag of specifically binding to a moiety which specifically recognizes the purification tag.

The present disclosure also relates to proteinaceous binding molecules comprising at least two variable domains, wherein one variable domain binds to a target molecule, and wherein the other variable domains are inert.

"Inert" as used herein in the context of the binding specificity of a variable domain refers to a variable domain which does not have binding specificity to another molecule. In particular, an inert variable domain does,not bind to a target molecule or any other molecule, and it does not comprise a purification tag. Proteinaceous binding molecules comprising at least two variable domains, wherein one variable domain binds to a target molecule, and wherein the other variable domains are inert, possess monovalent binding specificity for a target molecule and may be used in accordance with the present invention.

Also disclosed herein are proteinaceous binding molecules in which purification tags can be introduced into the variable domain. Preferably, said proteinaceous binding molecules do no longer bind to the target molecule of the original parental binders from which the VH and VL are derived. Alternatively, said proteinaceous binding molecule binds to the target molecule with at least 10-fold, at least 100-fold or at least 1000-fold less affinity as compared to the original parental binder from which the VH and VL are derived. Alternatively said proteinaceous binding molecule shows only residual binding affinity to the target molecule as compared to the original parental binder from which the VH and VL are derived. Methods and assays measure and compare affinities are known to the skilled artisan and are described in the experimental procedures section.

According to the present invention, a purification tag can be introduced into the variable domain, such as the variable domain of an immunoglobulin. The purification tag can be introduced into any CDR region of an immunoglobulin. The CDR regions of immunoglobulins are flanked by framework regions. The respective structures and boundaries of the CDR regions and framework regions are known to the person skilled in the art. The purification tag can be introduced into the variable domain in several different ways. The purification tag can be introduced into a CDR region in a way, so that the purification tag replaces the entire original CDR region. The purification tag can also be introduced in a way, so that the purification tag replaces one or more of the original amino acids of the original CDR region, but in a way that one or more of the original amino acids of the original CDR region are still present. The purification tag can also be introduced in a way, so that the purification tag is inserted into a CDR region, i.e. none of the original amino acids of the CDR region are deleted or replaced. The purification tag can also be introduced into the framework region of the variable domain, i.e. in a manner in which the respective variable domain does no longer bind to the target molecule after introduction of said purification tag, e.g. due to conformational changes. The purification tag can also be introduced into a region of the variable domain which overlaps the CDR region and the framework region. All such possibilities and options, as well as combinations thereof, will be self evident for the skilled artisan.

The present invention also relates to the DNA molecules that encode the proteinaceous binding molecules of the invention. DNA molecules of the invention are not limited to the sequences disclosed herein, but also include variants thereof. DNA variants within the invention may be described by reference to their physical properties in hybridization. The skilled worker will recognize that DNA can be used to identify its complement and, since DNA is double stranded, its equivalent or homolog, using nucleic acid hybridization techniques. It also will be recognized that hybridization can occur with less than 100% complementarity. However, given appropriate choice of conditions, hybridization techniques can be used to differentiate among DNA sequences based on their structural relatedness to a particular probe. For guidance regarding such conditions see, Sambrook et al., 1989 (Sambrook, J., Fritsch, E. F. and Maniatis, T. (1989) Molecular Cloning: A laboratory manual, Cold Spring Harbor Laboratory Press, Cold Spring Harbor, USA) and Ausubel et al., 1995 (Ausubel, F. M., Brent, R., Kingston, R. E., Moore, D. D., Sedman, J. G., Smith, J. A., & Struhl, K. eds. (1995). Current Protocols in Molecular Biology. New York: John Wiley and Sons).

The present invention further provides recombinant DNA constructs comprising one or more of the nucleotide sequences of the present invention. A recombinant construct of the present invention can be used in connection with a vector, such as a plasmid, phagemid, phage or viral vector, into which a DNA molecule encoding a proteinaceous binding molecule of the invention is inserted. The encoded gene may be produced by techniques described in Sambrook et al., 1989, and Ausubel et al., 1989. Alternatively, the DNA sequences may be chemically synthesized using, for example, synthesizers. See, for example, the techniques described in Oligonucleotide Synthesis (1984, Gait, ed., IRL Press, Oxford), which is incorporated by reference herein in its entirety. Recombinant constructs of the invention are comprised with expression vectors that are capable of expressing the RNA and/or protein products of the encoded DNA(s). The vector may further comprise regulatory sequences, including a promoter operably linked to the open reading frame (ORF). The vector may further comprise a selectable marker sequence. Specific initiation and bacterial secretory signals also may be required for efficient translation of inserted target gene coding sequences.

The present invention further provides host cells containing at least one of the DNA constructs of the present invention. The host cell can be virtually any cell for which expression vectors are available. It may be, for example, a higher eukaryotic host cell, such as a mammalian cell, a lower eukaryotic host cell, such as a yeast cell, and may be a prokaryotic cell, such as a bacterial cell. Introduction of the recombinant construct into the host cell can be effected, for example, by calcium phosphate transfection, DEAE, dextran mediated transfection, electroporation or phage infection.

In certain embodiments, the present invention provides proteinaceous binding molecules comprising at least two variable domains, wherein one variable domain binds to a target molecule, and wherein at least one other variable domain comprises a purification tag. The proteinaceous binding molecule may comprise two, three four or more than four variable domains. In preferred embodiments, the proteinaceous binding molecule comprises two variable domains.

According to the present invention, one variable domain of the proteinaceous binding molecule binds to a target molecule. None of the other variable domains binds to the target molecule, in particular the variable domain comprising the purification tag. In certain embodiments, the present invention relates to proteinaceous binding molecules comprising at least two variable domains, wherein the first variable domain binds to a target molecule and wherein at least one other variable domain comprises a purification tag suitable for purification of said proteinaceous binding molecule, and wherein none of said other variable domain binds to said target molecule. In other embodiments the proteinaceous binding molecule comprises at least two variable domains wherein the first variable domain binds to a target molecule, but none of said other variable domain binds to said target molecule.

In certain embodiments, the purification tag is selected from a histidine tag, a Strep tag, a myc tag, a V5 tag and a Flag tag, or a substantially identical variant thereof. In preferred embodiments, the purification tag is a histidine tag (His tag), most preferably a hexahistidine peptide. In alternative embodiments, the purification tag is a substantially identical variant of a histidine tag, such as a pentahistidine peptide or a heptahistine peptide. Other histidine tags with even more histidine residues, such as eight, nine, ten, or even more histidine residues, or polyhistidine stretches, in which one or more histidine residue is substituted to another amino acid, but which is still able to bind to Ni-NTA, may be used in accordance with the present invention as well.

In certain embodiments, the proteinaceous binding molecule is an immunoglobulin or a functional fragment thereof. In preferred embodiments, said immunoglobulin is an antibody, more preferably a full-length antibody.

In certain embodiments, the immunoglobulin is a human immunoglobulin. In alternative embodiments said immunoglobulin is a immunoglobulin from a rodent, such as a mouse or a rat.

In certain embodiments, the immunoglobulin is of any one of the classes IgG, IgM, IgE, IgA, and IgD. In preferred embodiments, the immunoglobulin is of the class IgG. In certain embodiments, the immunoglobulin is a human immunoglobulin of any one of the classes IgG, IgM, IgE, IgA, and IgD. In preferred embodiments, the immunoglobulin is a human immunoglobulin of the class IgG.

In certain embodiments, the immunoglobulin is of any one of the subclasses IgG1, IgG2, IgG3 and IgG4. In preferred embodiments, the immunoglobulin is of the subclass IgG1. In certain embodiments, the immunoglobulin is a human immunoglobulin of any one of the subclasses IgG1, IgG2, IgG3 and IgG4. In preferred embodiments, the immunoglobulin is a human immunoglobulin of the subclass IgG1.

In preferred embodiments the proteinaceous binding molecule is an immunoglobulin. In these embodiments the variable domains comprise a variable heavy (VH) chain and a variable light (VL) chain. In preferred embodiments the proteinaceous binding molecule is an immunoglobulin comprising two variable domains, each variable domain comprising a variable heavy (VH) chain and a variable light (VL) chain.

In certain embodiments, the variable heavy (VH) chain comprises three complementary determining regions, H-CDRs. In preferred embodiments the proteinaceous binding molecule is an immunoglobulin comprising a variable heavy (VH) chain comprisings three complementary determining regions, H-CDR1, H-CDR2 and H-CDR3.

In certain embodiments, the variable light (VH) chain comprises three complementary determining regions, L-CDRs. In preferred embodiments the proteinaceous binding molecule is an immunoglobulin comprising a variable light (VH) chain comprisings three complementary determining regions, L-CDR1, L-CDR2 and L-CDR3.

In certain embodiments, the purification tag is comprised in the variable heavy (VH) chain or the variable light (VL) chain of the proteinaceous binding molecule or immunoglobulin. In preferred embodiments, said purification tag is comprised in the variable heavy (VH) chain of the proteinaceous binding molecule or immunoglobulin. In more preferred embodiments the purification tag is comprised in any one of the three complementary determining regions (H-CDRs) of the variable heavy (VH) chain. In even more preferred embodiments the purification tag is comprised in the H-CDR3, the H-CDR2 or the H-CDR1, more preferrably the H-CDR3 or the H-CDR2 and most preferrably the H-CDR3.

In certain embodiments, the purification tag is comprised in the variable light (VL) chain of the proteinaceous binding molecule or immunoglobulin. In more preferred embodiments, the purification tag is comprised in any one of the three complementary determining regions (L-CDRs) of the variable light (VL) chain. In even more preferred embodiments, the purification tag is comprised in the L-CDR3, the L-CDR2 or the L-CDR1, and in most preferred embodiments in the L-CDR3.

The term "CDR region" refers to the regions of an immunoglobulin that typically contain any complementary determining region. The complementary determining regions of an immunoglobulin are comprised in the variable heavy (VH) chain and the variable light (VL) chain. Specifically, the complementary determining regions of an immunoglobulin are the H-CDR1, the H-CDR2, the H-CDR3, the L-CDR1, the L-CDR2 or the L-CDR3 region.

The present invention further provides nucleic acid sequences that encode the proteinaceous binding molecules of the present invention. Proteinaceous binding molecules of the present invention may consist of one, two, three four, or even more polypeptides. Each of said polypeptides may be encoded by the same or by different nucleic acids. Likewise, the nucleic acids encoding said individual peptides of a proteinaceous binding molecule of the present invention may be present on the same or on different vectors.

The present invention further provides nucleic acid sequences that encode a variable domain comprising a purification tag. Preferably, said variable domain is comprised in an immunoglobulin or a functional fragment thereof. In certain embodiments, said variable domain is comprised in the variable heavy (VH) chain or the variable light (VL) chain of said immunoglobulin, preferably in the variable heavy (VH) chain, and more preferably in the H-CDR3 of said variable heavy (VH) chain.

The present invention further provides vectors comprising the nucleic acid sequences of the proteinaceous binding molecules or comprising the nucleic acid sequences of variable domains comprising a purification tag according to the present invention.

The present invention further provides cells and host cells comprising said vectors. Preferably said cells or host cells are mammalian cells. Also preferably said cells or host cells are isolated cells or isolated host cells.

The present invention further provides pharmaceutical compositions comprising a proteinaceous binding molecule of the present invention and a pharmaceutical acceptable carrier or excipient therefor.

Also disclosed herein are therapeutic methods for treating a disease or disorder comprising the step of administering to a subject in need thereof a therapeutically effective amount of the pharmaceutical composition comprising a proteinaceous binding molecule of the present invention and a pharmaceutical acceptable carrier or excipient therefor. Preferably said disease or disorder is associated with the undesired presence of the target molecule.

A "therapeutically effective" amount hereby is defined as the amount of a proteinaceous binding molecule that alone, or in combination with other therapies, provides a therapeutic benefit in the treatment or management of an adverse condition. The term can encompass an amount that improves overall therapy, reduces or avoids unwanted effects, or enhances the therapeutic efficacy of or synergies with another therapeutic agent. The "subject" in need of a treatment may be a human or non-human animal (e.g., rabbit, rat, mouse, monkey or other lower-order primate).

A proteinaceous binding molecule of the disclosure might be co-administered with known medicaments, and in some instances the proteinaceous binding molecule might itself be modified. For example, a proteinaceous binding molecule could be conjugated to an immunotoxin or radioisotope to potentially further increase efficacy.

The proteinaceous binding molecules can be used as a therapeutic or a diagnostic tool in a variety of situations where the target molecule is undesirably expressed or found. To treat any diseases or disorders, pharmaceutical compositions for use in accordance with the present invention may be formulated in a conventional manner using one or more physiologically acceptable carriers or excipients. A proteinaceous binding molecule of the invention can be administered by any suitable means, which can vary, depending on the type of disorder being treated. Possible administration routes include parenteral (e.g., intramuscular, intravenous, intraarterial, intraperitoneal, or subcutaneous), intrapulmonary and intranasal, and, if desired for local immunosuppressive treatment, intralesional administration. In addition, a proteinaceous binding molecule of the invention might be administered by pulse infusion, with, e.g., declining doses of the proteinaceous binding molecule. Preferably, the dosing is given by injections, most preferably intravenous or subcutaneous injections, depending in part on whether the administration is brief or chronic. The amount to be administered will depend on a variety of factors such as the clinical symptoms, weight of the individual, whether other drugs are administered. The skilled artisan will recognize that the route of administration will vary depending on the disorder or condition to be treated.

Determining a therapeutically effective amount of the proteinaceous binding molecule, according to this invention, largely will depend on particular patient characteristics, route of administration, and the nature of the disorder being treated. General guidance can be found, for example, in the publications of the International Conference on Harmonisation and in Remington's Pharmaceutical Sciences, chapters 27 and 28, pp. 484-528 (18th ed., Alfonso R. Gennaro, Ed., Easton, Pa.: Mack Pub. Co., 1990). More specifically, determining a therapeutically effective amount will depend on such factors as toxicity and efficacy of the medicament. Toxicity may be determined using methods well known in the art and found in the foregoing references. Efficacy may be determined utilizing the same guidance.

Also described herein are methods for detecting a target molecule in a subject or a sample, comprising the step of contacting said subject or sample with a proteinaceous binding molecule of the present invention.

Also disclosed herein are methods for diagnosing a disease or disorder in a subject, comprising the step of contacting said subject or sample with a proteinaceous binding molecule of the present invention.

Also disclosed herein is the use of a proteinaceous binding molecule of the present invention for the manufacture of a medicament.

The present invention further provides a proteinaceous binding molecule of the present invention for the treatment of a disease or disorder.

The present invention further provides the use of a proteinaceous binding molecule of the present invention for the diagnosis of a disease or disorder.

Also disclosed herein is the use of a proteinaceous binding molecule of the present invention for the detection of a target molecule.

In certain embodiments the proteinaceous binding molecule is an immunoglobulin, such as an antibody. In such cases, the cell line transfected with the nucleic acid molecules of the present invention produces two different variable heavy (VH) chains - one VH chain which is part of the variable domain which binds to the target molecule (hereinafter "VH-targ"), and one VH chain which is part of the variable domain which comprises the purification tag (hereinafter "VH-puri"; alternatively this VH chain may be inert). Only one variable light (VL) chain is produced. This leads to the production of three different immunoglobulin species:
- Species A comprises two variable heavy (VH) chains of the type VH-targ.
- Species B comprises one variable heavy (VH) chain of the type VH-targ and one variable heavy (VH) chain of the type VH-puri.
- Species C comprises two variable heavy (VH) chains of the type VH-puri.

All immunoglobulin species comprise two identical variable light (VL) chains.

Species A of the immunoglobulins described above does not comprise a purification tag. Species B comprises one purification tag, species C comprises two purification tags. Species B is the species with the desired properties according to the present invention, i.e. monovalent binding of the target molecule.

Species B and C can both be purified via a moiety which has affinity for the purification tag, e.g. via Ni-NTA, if the purification tag is a His tag. Since the avidity of immunoglobulin species C is much higher compared to species B due to the presence of a second purification tag, these species can be conveniently separated via appropriate purification steps. Possible purification techniques include chromatography, such as column chromatography. Elution can be accomplished by various ways. Preferably a gradient is applied. Respective techniques are known to the skilled artisan. If a histidine tag is used, elution is most conveniently achieved by way of a imidazole gradient. The process is represented in Figure 1. Due to its higher avidity, species C has higher affinity to the column, and hence higher concentrations of a solution, such as a solution comprising imidazole, will be needed to recover said species from the chromatography column. By way of this technique, i.e. a higher imidazole concentration for elution, species B can be conveniently separated from species C.

Therefore, the present invention provides a method to purify a proteinaceous binding molecule of the present invention, comprising the steps of (a) providing a solution comprising a proteinaceous binding molecule of the present invention, (b) bringing the solution of step (a) in contact with a moiety with affinity to the purification tag of said proteinaceous binding molecule, (c) allowing the binding of the proteinaceous binding molecule to said moiety, (d) removing all or substantially all constituents of the solution which do not bind to the moiety, and (e) recovering the proteinaceous binding molecule from the moiety.

The solution comprising a proteinaceous binding molecule of the present invention is preferably the culture supernatant of a cell expressing said proteinaceous binding molecule. Alternatively, any other solution comprising a proteinaceous binding molecule according to the present invention may be used.

In another aspect the solution comprising a proteinaceous binding molecule of the present invention may further comprise a proteinaceous binding molecules comprising two purification tags.

Preferably, the proteinaceous binding molecule of the present invention is separated from a solution comprising proteinaceous binding molecules comprising two purification tags via a gradient, preferably a imidazole gradient.

In certain aspects, the present disclsure provides a method to separate a first proteinaceous binding molecule comprising at least two variable domains, wherein one variable domain binds to a target molecule, and wherein one other variable domain comprises a purification tag from a second proteinaceous binding molecule comprising two purification tags, comprising the steps of (a) providing a solution comprising a the first and the second proteinaceous binding molecule, (b) bringing the solution of step (a) in contact with a moiety with affinity to the purification tag of said proteinaceous binding molecules, (c) allowing the binding of the proteinaceous binding molecules to said moiety, (d) removing all or substantially all constituents of the solution which do not bind to the moiety, and (e) recovering the proteinaceous binding molecules from the moiety in a manner that the first proteinaceous binding molecule is separated from the second proteinaceous binding molecule. In preferred embodiments step (e) comprises the application of a gradient to recover the proteinaceous binding molecule from the moiety with affinity for the purification tag. If the purification tag is a histidine tag, then an imidazole gradient is preferred.

In certain embodiments, the present invention provides an immunoglobulin, or a functional fragment thereof, comprising at least two variable domains, wherein one variable domain binds to a target molecule, and wherein at least one other variable domain comprises a purification tag, and wherein said purification tag is comprised in the CDR region of said other variable domain. Most preferred said immunoglobulin is a full length immunoglobulin.

In certain embodiments, the present invention provides proteinaceous binding molecules comprising at least two variable domains, wherein one variable domain binds to a target molecule, and wherein at least one other variable domain comprises a purification tag or is inert. In certain embodiments, said proteinaceous binding molecules may comprise two variable domains. In certain embodiments, the variable domain comprising the purification tag does not bind to the target molecule. In certain embodiments, the purification tag is selected from a histidine tag, a Strep tag, a myc tag, a Flag tag, a V5 tag, and a substantially identical variant of the foregoing. In certain embodiments, the purification tag is a histidine tag or a substantially identical variant thereof. In certain embodiments, the histidine tag is a hexahistidine peptide. In certain embodiments, the proteinaceous binding molecule is an immunoglobulin or a functional fragment thereof. In certain embodiments, the immunoglobulin is an antibody. In certain embodiments, the antibody is a human, an humanized or a chimeric antibody. In certain embodiments, the antibody is a human antibody. In certain embodiments, the human antibody is of the class IgG. In certain embodiments, the human antibody is of any one of the subclasses IgG1, IgG2, IgG3 and IgG4. In certain embodiments, the human antibody is of the subclass IgG1. In certain embodiments, each of said variable domains comprises a variable heavy (VH) chain and a variable light (VL) chain. In certain embodiments, the variable heavy (VH) chain comprises three complementary determining regions (H-CDRs). In certain embodiments, the variable light (VL) chain comprises three complementary determining regions (L-CDRs). In certain embodiments, the purification tag is comprised in the variable heavy (VH) chain or the variable light (VL) chain. In certain embodiments, the purification tag is comprised in the variable heavy (VH) chain. In certain embodiments, the purification tag is comprised in any one of the three complementary determining regions (H-CDRs) of the variable heavy (VH) chain. In other embodiments, the purification tag is comprised in more than one of the three complementary determining regions (H-CDRs) of the variable heavy (VH) chain. In certain embodiments, the purification tag is comprised in the H-CDR3 or the H-CDR2. In certain embodiments, the purification tag is comprised in the H-CDR3. In certain embodiments, the purification tag is comprised in the variable light (VL) chain. In certain embodiments, the purification tag is comprised in any one of the three complementary determining regions (L-CDRs) of the variable light (VL) chain. In other embodiments, the purification tag is comprised in more than one of the three complementary determining regions (L-CDRs) of the variable light (VL) chain. In certain embodiments, the purification tag is comprised in the L-CDR2.

In certain embodiments, the present invention provides nucleic acid sequences that encode a proteinaceous binding molecule according to the present invention, including the proteinaceous binding molecules recited in the foregoing paragraph. In certain embodiments, these nucleic acid sequences encode a variable domain comprising a purification tag. In certain embodiments, the variable domain is comprised in an immunoglobulin or a functional fragment thereof. In certain embodiments, the variable domain of these nucleic acid sequences is comprised in the variable heavy (VH) chain or the variable light (VL) chain of said immunoglobulin. In certain embodiments, the variable domain of these nucleic acid sequences is comprised in the variable heavy (VH) chain of said immunoglobulin. In certain embodiments, the variable domain of these nucleic acid sequences is comprised in the H-CDR3 of said variable heavy (VH) chain.

In certain embodiments the present invention provides a vector comprising a nucleic acid sequence of the preceding paragraph.

In certain embodiments, the present invention provides a cell comprising a vector of the present invention, including the vectors of the preceding paragraph. In certain embodiments the cell is a mammalian cell. In certain embodiments the cell is an isolated cell.

In certain embodiments the present invention provides pharmaceutical compositions comprising a proteinaceous binding molecule as described hereinabove and a pharmaceutical acceptable carrier or excipient therefor.

In certain embodiments the present invention provides a method for treating a disease or disorder, comprising the step of administering to a subject in need thereof a therapeutically effective amount of the pharmaceutical composition of the present invention, including the pharmaceutical compositions of the preceding paragraph. In certain embodiments, the disease or disorder is associated with the undesired presence of the target molecule.

In certain embodiments, the present invention provides proteinaceous binding molecules as described hereinabove for the treatment of a disease or disorder.

In certain embodiments, the present invention provides the use of a proteinaceous binding molecule as described hereinabove for the diagnosis of a disease or disorder.

In certain embodiments, the present invention provides the use of a proteinaceous binding molecule as described hereinabove for the detection of a target molecule.

In certain embodiments, the present invention provides a method to purify a proteinaceous binding molecule as described hereinabove comprising the steps of (a) providing a solution comprising a proteinaceous binding, (b) bringing the solution of step (a) in contact with a moiety with affinity to the purification tag of said proteinaceous binding molecule, (c) allowing the binding of the proteinaceous binding molecule to said moiety, (d) removing all or substantially all constituents of the solution which do not bind to the moiety, and (e) recovering the proteinaceous binding molecule from the moiety. In certain embodiments the solution further contains a proteinaceous binding molecules comprising two purification tags. In certain embodiments the proteinaceous binding molecule comprising one purification tag is separated from the proteinaceous binding molecules comprising two purification tags via a gradient. In certain embodiments the gradient is an imidazole gradient.

### Examples

### Example 1: Generation of Fab fragments containing a purification tag in the H-CDR3 region

MOR03207_FS is a Fab with specificity for hen egg lysozyme. It comprises a Flag tag and a Strep tag at the C-terminus of the VH chain, i.e. outside the variable domain. Via appropriate primer design and conventional cloning techniques, a hexahistidine tag is introduced into the H-CDR3 of the variable heavy chain of the encoding expression vector pMx9_MOR03207_FS (Rauchenberger et al, 2003), yielding pMx9_MOR08428, which encodes following H-CDR3 sequence: GYSGHHHHHHSGDY. MOR08428 comprises a Flag tag and a Strep tag (FS) at the C-terminus of the VH chain.

Test purifications confirm that both MOR03207_FS and MOR08428_FS can be purified via the Strep tag. However, MOR08428_FS could be alternatively purified by Immobilised Metal Ion Affinity Chromatography (IMAC), confirming that the hexahistidine tag is freely accessible and therefore can be utilized for affinity chromatography purposes. Finally, via ELISA tests it was confirmed that MOR03207_FS, but not MOR08428_FS, is able to bind to hen egg lysozyme, indicating that the specificity of the original Fab fragment was destroyed by way of incorporation of the hexahistidine tag into H-CDR3 (Figure 2).

Next, the VH of MOR08428_FS was combined by cloning with the VL of MOR03080. MOR03080 is a Fab with specificity for human CD38. The yielding construct, designated MOR08441_FS, hence contains the VH of MOR08428 and the VL of MOR03080.

Via FACS analysis (Figure 3) and ELISA tests (Figure 2) it was confirmed that MOR03080, but neither MOR08428 nor MOR08441, is able to bind to human CD38. Likewise, MOR03207_FS, but neither MOR08428 nor MOR08441, is able to bind to hen egg lysozyme. Finally, MOR08428_FS can be purified by Ni-NTA chromatography, showing that the 6His sequence incorporated in the H-CDR3 is accessible for the affinity column matrix.

In summary, it is demonstrated that via the incorporation of a purification tag, such as a hexahistidine tag, into the variable domain of a proteinaceous binding molecule, such as incorporation of a hexahistidine tag into H-CDR3 of a human Fab fragment, it is possible to generate Fabs which (a) can be easily purified by way of their binding to respective affinity resins, and (b) are no longer able to bind to the target molecule of the original parental binders, where VH and VL was derived from.

### Example 2: Generation of full-length IgG's containing a purification tag in the H-CDR3 region

Monovalent immunoglobulins of the present invention can be generated as described in this example. Alternatively, other techniques may be employed. For example certain experimental techniques exist, which allow the favourable pairing of the desired immunoglobulin heavy chains. For example, bispecific IgG's can be generated by a technology using engineered disulfide bonds and "knob into holes" mutations (Merchant et al, Nature Biotechnology (1998) 16, 677), enabling the efficient pairing of the desired heavy chains. Other related techniques known in the art may be employed as well.

In the following, Fab fragments of Example 1 are converted into IgG1 format. Therefore, the respective constructs are cloned into appropriate vectors.

Variable heavy chains of MOR08428 and MOR03080 were cloned into pMorph2_hulgG1, a vector harbouring the constant human gamma1 constant region, thereby generating vectors pMorph2_h_IgG1_MOR08428 and pMorph2_h_IgG1_MOR03080, respectively. The variable light chain of of MOR03080 was cloned into pMorph2_h_Ig_lambda2, a vector harbouring the constant human lambda light chain, yielding in vector pMorph2_h_Ig_lambda2_MOR03080. All three constructs are co-transfected and expressed in a human cell line, such as HKB11 (ATCC number: CRL-12568; Cho et al. J Biomed Sci. (2002):631-8). The cell culture supernatant is used for further experiments. The cell culture supernatant was prepurifed via standard Protein A affinity chromatography.

Since two different heavy chains and one light chain are produced in the transfected cell line, altogether three different immunoglobulin species are produced. One species contains two heavy chains of MOR03080. This immunoglobulin species does not contain a hexahistine tag and hence has no affinity to Ni-NTA and no monovalent, but bivalent specificity for CD38. The second immunoglobulin species contains one heavy chain of MOR03080 and one heavy chain of MOR08428. This immunoglobulin species is the species with the desired properties according to the present invention. It has affinity to Ni-NTA, due to its hexahistine Tag in H-CDR3 of the single MOR08428 heavy chain. Finally, the third immunoglobulin species contains two heavy chains of MOR08428. This species has no affinity to the target molecule (in this case human CD38). However, this species contains a hexahistidine tag on both arms of the immunoglobulin and therefore shows bivalent binding to the resin of IMAC column (e.g. Ni-NTA (Qiagen) or His-Bind Fractogel (Merck)), hence having higher avidity for the resin. Since the avidity to the resin of this species is much higher than the monovalent affinity of the second, desired, immunoglobulin species, it can be conveniently separated via affinity chromatography, e.g. via a imidazole gradient. This elution process is represented in Figure 1.

This Protein A purified mixture of IgG1 molecules was subjected to IMAC purification. A His-Bind Fractogel (Merck #70693-3) column was loaded with the pre-purified IgG1 mixture on a Akta-FPLC system. At a flow-rate of 1 ml/min 1 ml fractions were taken.

As running buffer 20 mM Na-phosphate pH 7.4/500 mM NaCl/10 mM imidazole was used. As elution buffer running buffer + 250 mM imidazole was used, while a gradient: 0 - 100% elution buffer was applied. Representative fractions were loaded on a reducing analytical SDS PAGE (Figure 4). As expected a single light chain band was observed at ∼30 KD. In contrast two distinct heavy chain bands could be distinguished (both - 50 KD). The lower band was observed in the column flow through, while the upper band only appeared upon elution with imidazole. At high imidazole concentrations (i.e. later fractions) only the upper heavy chain band was eluted. It is concluded that the lower heavy chain band represents the heavy chain of MOR03080, while the upper band represents the heavy chain of MOR08428, carrying the hexahistidine tag.

Therefore, untagged (bivalent) MOR03080 IgG1 could be easily separated by hexahistidine tagged IgG species. Furthermore, the elution by an imidazole gradient enabled a further separation of single hexahistidine tagged and double hexahistidine tagged IgG1 species.

### Example 3: Functional analysis of separated IgG1 species

Fractions #30-58 and fractions #70-90 were pooled (see Figure 4). The buffer of these pooled fractions, as well as of the column flow through, was exchanged to PBS via dialysis. Protein concentrations of these samples were determined by A280 nm measurement. These three samples were analyzed in SDS-PAGE under reducing conditions (Figure 5). The heavy chain of MOR03080 has a lower apparent molecular weight compared to the heavy chain of MOR08428. The flow through sample contained only the heavy chain of MOR03080, while the pooled sample from fractions #70-90 predominantly contained the heavy chain of MOR08428. In contrast the pooled sample from #30-58 contained an approximate 1:1 ratio of both heavy chains. These data show that bivalent IgG1 was separated from the single hexahistidine tagged monovalent IgG1 and double hexahistidine tagged IgG1 by using affinity chromatography while applying an imidazole gradient for elution.

### EC₅₀ determination in FACS

To characterize the binding properties of these IgG1 fractions FACS EC₅₀ determinations were perfomed on CD38 expressing RPMI8226 cell. To this end IgGs at defined concentration (250 nM to 0.24 pM) were used as primary antibody. Detection was performed using 50 µl/well of 1:200 diluted PE-conjugated secondary antibody (goat anti-human IgG ((Fab)'₂ fragment specific); JIRLaboratories #109-116-097). EC₅₀ values were determined using Prism 3.03 software (GraphPad Software) applying a non-linear regression curve fit model (sigmoidal dose response; variable slope). Table 1 shows EC50 values obtained in this FACS titration experiment.

| IgG | EC₅₀ (nM) |
|---|---|
| MOR03080 IgG1 | 0.4 |
| MOR03080 Fab | 1.2 |
| Flow through | 0.5 |
| Fraction # 30-58 IgG1 | 1.5 |
| Fraction # 70-90 IgG1 | 13.4 |
| MOR08588 IgG1 | No binding |
| MOR03207 IgG1 | No binding |

The EC50 value of the flow through IgG1 fraction was very similar to the MOR03080 reference IgG1, showing that the flow through consistet of bivalent CD38 binding IgG1. In contrast, fraction #30-58 showed a three fold lower EC50 (1.5 nM), being in the same range as the monovalent reference Fab fragment of MOR03080. This indicates that the IgG1 fraction #30-58 consisted of monovalent CD38 binding IgG1. Reference IgG1 MOR08588, which solely consists of the VH of MOR08428 and the VL of MOR03080 is therefore double hexahistidine tagged and does not bind to CD38, as expected. Fraction #70-90, which was eluted at high imidazole concentrations is mainly made of the double hexahistidine tagged IgG1 species, as evidenced by the 10fold lower EC50 compared to the EC50 of fraction #30-58. The residual binding properties are attributable to samples impurities of co-eluted single hexahistidine tagged IgG1 species.

### Affinity determination by plasmon surface resonance (Biacore)

The kinetic constants kₒₙ and k_{off} were determined with serial dilutions of the respective purified antibodies binding to covalently immobilized human CD38-Fc using the Biacore 3000 instrument (Biacore, Uppsala, Sweden). Covalent antigen immobilization was achieved by a standard EDC-NHS coupling procedure. Kinetic measurements were done in PBS, pH 7.2 at a flow rate of 20 µl/min using antibody concentration ranging from 1.5-500 nM. Injection time for each concentration was 1 min, followed by 3 min dissociation phase. For regeneration two injections of 5 µl 10mM Glycine buffer, pH 1.5 were used. All sensograms were fitted using the BIA evaluation software 3.2 (Biacore), assuming a 1:1 binding kinetics.

**Table 2 shows apparent affinity values obtained from the Biacore experiment**

| Antibody | Rₘₐₓ | apparent kₒₙ | apparent k_{off} | apparent K_{D} (nM) |
|---|---|---|---|---|
| MOR03080 IgG1 | 275 | 9.13E+04 | 2.60E-04 | **3** |
| MOR03080 Fab | 100 | 6.07E+04 | 1.64E-03 | **27** |
| Flow through | 182 | 9.80E+04 | 2.84E-04 | **3** |
| Fraction # 30-58 IgG1 | 140 | 4.46E+04 | 1.39E-03 | **31** |
| Fraction # 70-90 IgG1 | 44 | 2.80E+04 | 1.38E-03 | **49** |
| MOR08588 IgG1 | - | - | - | No binding |

The apparent K_{D} of the flow through sample was very similar to purified MOR03080 IgG1, indicating that this sample consists of bivalent IgG1. In contrast the pooled sample from #30-58 showed an apparent K_{D} very similar to the MOR03080 Fab fragment, which is tenfold lower compared to MOR03080 IgG1. This result indicates that the pooled sample from fraction #30-58 show the same monovalent binding properties as the MOR03080 Fab fragment. MOR08588 IgG1 did not show any binding to CD38-Fc, as expected. Fraction # 70-90 IgG1 however did show binding to CD38-Fc. Fraction #70-90, which was eluted at high imidazole concentrations should be mainly made of the double hexahistidine tagged IgG1 species. This is illustrated by the lower Rₘₐₓ compared to e.g. fraction #30-58. The Kₒₙ and k_{off} observed in these samples are most likely attributable to samples impurities of co-eluted single hexahistidine tagged IgG1 species.

In summary, full-length immunoglobulins with monovalent affinity for a target molecule are produced. These molecules can be easily produced and purified via Ni-NTA chromatography employing the purification tag, thereby, separating them from bivalently binding immunoglobulin molecules carrying no such purification tag.

## Claims

1. An immunoglobulin comprising at least two variable domains, wherein a variable domain includes the entire antigen-binding region of one arm of the immunoglobulin molecule, wherein one variable domain binds to a target molecule, and wherein at least one other variable domain comprises a purification tag, wherein the variable domain comprising the purification tag does not bind to the target molecule, and wherein said purification tag is comprised in the CDR region of said other variable domain and is selected from a histidine tag, a hexahistidine tag, a Strep tag, a myc tag, a Flag tag and a V5 tag, or a substantially identical variant thereof which retains the property of the purification tag of specifically binding to a moiety which specifically recognizes the purification tag and wherein the immunoglobulin is monovalent with respect to binding of the target molecule but retains the properties of a full-length immunoglobulin in respect of size and presence of the Fc domain.

2. The immunoglobulin according to claim 1, wherein said purification tag is comprised in the H-CDR3 or the H-CDR2.

3. The immunoglobulin, according to any one of claims 1 to 2, wherein said purification tag is comprised in the L-CDR2.

4. A nucleic acid sequence that encodes an immunoglobulin of any one of the preceding claims.

5. A vector comprising a nucleic acid sequence of claim 4.

6. A cell comprising a vector according to claim 5.

7. The cell according to claim 6, wherein said cell is a mammalian cell.

8. A pharmaceutical composition comprising an immunoglobulin, of any one of claims 1 to 3 and a pharmaceutical acceptable carrier or excipient therefor.

9. An immunoglobulin according to any one of claims 1 to 3 for use in the diagnosis of a disease or disorder.

10. An immunoglobulin according to any one of claims 1 to 3 for use in the treatment of a disease or disorder.

11. A method to purify an immunoglobulin, comprising the steps of (a) providing a solution comprising an immunoglobulin according to any one of claims 1 to 3, (b) bringing the solution of step (a) in contact with a moiety with affinity to the purification tag of said immunoglobulin, (c) allowing the binding of the immunoglobulin to said moiety, (d) removing all or substantially all constituents of the solution which do not bind to the moiety, and (e) recovering the immunoglobulin from the moiety.

## Patentansprüche

1. Immunglobulin, das mindestens zwei variable Domänen umfasst, wobei eine variable Domäne die gesamte Antigenbindungsregion von einem Arm des Immunglobulin-moleküls umfasst, wobei eine variable Domäne an ein Zielmolekül bindet, und wobei mindestens eine weitere variable Domäne ein Aufreinigungs-Tag umfasst, wobei die variable Domäne, die das Aufreinigungs-Tag umfasst, nicht an das Zielmolekül bindet, und wobei das Aufreinigungs-Tag in der CDR-Region der anderen variable Domäne umfasst ist und aus einem Histidin-Tag, einem Hexahistidin-Tag, einem Strep-Tag, einem myc-Tag, einem Flag-Tag und einem V5-Tag ausgewählt ist, oder eine im Wesentlichen identische Variante davon, die die Eigenschaft des Aufreinigungs-Tags, spezifisch an einen Molekülteil zu binden, der das Aufreinigungs-Tag spezifisch erkennt, beibehält, und wobei das Immunglobulin in Bezug auf die Bindung des Zielmoleküls monovalent ist, jedoch die Eigenschaften eines Volllängen-Immunglobulins in Bezug auf Größe und Vorliegens der Fc-Domäne beibehält.

2. Immunglobulin nach Anspruch 1, wobei das Aufreinigungs-Tag in der H-CDR3 oder der HCDR2 umfasst ist.

3. Immunglobulin nach einem der Ansprüche 1 bis 2, wobei das Aufreinigungs-Tag in der L-CDR2 umfasst ist.

4. Nukleinsäuresequenz, die für ein Immunglobulin nach einem der vorhergehenden Ansprüche codiert.

5. Vektor, der eine Nukleinsäuresequenz nach Anspruch 4 umfasst.

6. Zelle, die einen Vektor nach Anspruch 5 umfasst.

7. Zelle nach Anspruch 6, wobei es sich bei der Zelle um eine Säugetierzelle handelt.

8. Pharmazeutische Zusammensetzung, die ein Immunglobulin nach einem der Ansprüche 1 bis 3 und einen pharmazeutisch unbedenklichen Träger oder Exzipienten hierfür umfasst.

9. Immunglobulin nach einem der Ansprüche 1 bis 3 zur Verwendung in der Diagnose einer Krankheit oder eines Leidens.

10. Immunglobulin nach einem der Ansprüche 1 bis 3 zur Verwendung in der Behandlung einer Krankheit oder eines Leidens.

11. Verfahren zur Aufreinigung eine Immunglobulins, das die folgenden Schritte umfasst: (a) Bereitstellen einer Lösung, die ein Immunglobulin nach einem der Ansprüche 1 bis 3 umfasst, (b) Inkontaktbringen der Lösung von Schritt (a) mit einem Molekülteil mit Affinität für das Aufreinigungs-Tag des Immunglobulins, (c) Bindenlassen des Immunglobulins an den Molekülteil, (d) Entfernen von allen oder im Wesentlichen allen Bestandteilen der Lösung, die nicht an den Molekülteil binden, sowie (e) Gewinnen des Immunglobulins von dem Molekülteil.

## Revendications

1. Immunoglobuline comprenant au moins deux domaines variables, dans laquelle un domaine variable comprend la région complète de liaison à l'antigène de l'un des bras de la molécule d'immunoglobuline, dans laquelle un domaine variable se lie à une molécule cible et dans laquelle au moins un autre domaine variable comprend une étiquette de purification, dans laquelle le domaine variable comprenant l'étiquette de purification ne se lie pas à la molécule cible et dans laquelle ladite étiquette de purification est comprise dans la région CDR dudit autre domaine variable et est choisie parmi une étiquette d'histidine, une étiquette d'hexahistidine, une étiquette Strep, une étiquette myc, une étiquette Flag et une étiquette V5 ou un variant substantiellement identique à celles-ci, qui conserve la propriété de l'étiquette de purification de se lier spécifiquement à une fraction qui reconnaît spécifiquement l'étiquette de purification et dans laquelle l'immunoglobuline est monovalente en ce qui concerne la liaison de la molécule cible, mais elle conserve les propriétés d'une immunoglobuline de longueur totale quant à la taille et à la présence du domaine Fc.

2. Immunoglobuline selon la revendication 1, dans laquelle ladite étiquette de purification est comprise dans la H-CDR3 ou la H-CDR2.

3. Immunoglobuline selon l'une quelconque des revendications 1 à 2, dans laquelle ladite étiquette de purification est comprise dans la L-CDR2.

4. Séquence d'acide nucléique qui code pour une immunoglobuline selon l'une quelconque des revendications précédentes.

5. Vecteur comprenant une séquence d'acide nucléique selon la revendication 4.

6. Cellule comprenant un vecteur selon la revendication 5.

7. Cellule selon la revendication 6, dans laquelle ladite cellule est une cellule de mammifère.

8. Composition pharmaceutique comprenant une immunoglobuline, selon l'une quelconque des revendications 1 à 3, ainsi qu'un véhicule ou excipient pour celle-ci acceptable d'un point de vue pharmaceutique.

9. Immunoglobuline, selon l'une quelconque des revendications 1 à 3, destinée à être utilisée dans le diagnostic d'une maladie ou d'un trouble.

10. Immunoglobuline, selon l'une quelconque des revendications 1 à 3, destinée à être utilisée dans le traitement d'une maladie ou d'un trouble.

11. Procédé de purification d'une immunoglobuline, comprenant les étapes consistant à (a) fournir une solution comprenant une immunoglobuline, selon l'une quelconque des revendications 1 à 3, (b) mettre en contact la solution de l'étape (a) avec une fraction ayant une affinité pour l'étiquette de purification de ladite immunoglobuline, (c) permettre qu'ait lieu la liaison de l'immunoglobuline à ladite fraction, (d) ôter tous ou substantiellement tous les constituants de la solution qui ne se lient pas à la fraction, et (e) récupérer l'immunoglobuline à partir de la fraction.
